(19) Europäisches Patentamt
European Patent Office
Office européen des brevets

(11) Veröffentlichungsnummer: **0 018 586**
**B2**

# (12) NEUE EUROPÄISCHE PATENTSCHRIFT

(45) Veröffentlichungstag der neuen Patentschrift: **07.01.87**

(51) Int. Cl.⁴: **C 07 C 125/073**, C 07 C 125/077, C 07 D 295/12 // C07D239/04

(21) Anmeldenummer: **80102182.5**

(22) Anmeldetag: **23.04.80**

(54) **Verfahren zur Herstellung von aliphatischen und cycloaliphatischen Di- und Polyurethanen.**

(30) Priorität: **30.04.79 DE 2917493**

(43) Veröffentlichungstag der Anmeldung:
**12.11.80 Patentblatt 80/23**

(45) Bekanntmachung des Hinweises auf die Patenterteilung:
**08.12.82 Patentblatt 82/49**

(45) Bekanntmachung des Hinweises auf die Entscheidung über den Einspruch:
**07.01.87 Patentblatt 87/2**

(84) Benannte Vertragsstaaten:
**AT BE CH DE FR GB IT LI NL SE**

(56) Entgegenhaltungen:
**EP - A - 0 066 231**
**DE - A - 2 943 551**
**JP - A - 38 010 748**
**US - A - 2 409 712**
**US - A - 2 806 051**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

**EP 0 018 586 B2**

**Beschreibung**

Die Erfindung betrifft ein Verfahren zur Herstellung von aliphatischen und/oder cycloaliphatischen Di- und/oder Polyurethanen aus primären aliphatischen und/oder cycloaliphatischen Di- und/oder Polyaminen, Harnstoff und Alkoholen bei erhöhten Temperaturen gegebenenfalls in Gegenwart von Katalysatoren.

N-substituierte Urethane werden technisch üblicherweise durch Umsetzung von Alkoholen mit Isocyanaten oder von Aminen mit Chlorkohlensäureester hergestellt, wobei sowohl die Isocyanate als auch die Chlorkohlensäureester durch Phosgenierung der Alkohole gewonnen werden (Methoden der organischen Chemie, Houben—Weyl, Band 8, Seiten 137, 120 und 101, Georg Thieme Verlag, Stuttgart, 1952). Diese Verfahren sind technisch sehr aufwendig; ferner bringt die Verwendung von Phosgen wegen damit verbundener Sicherheits- und Umweltschutzmassnahmen erhebliche Nachteile mit sich.

N-substituierte Urethane finden als Zwischen- und Endprodukte Verwendung, Gemäss DE-OS 2 635 490 oder US-PS 3 919 278 sind sie beispielsweise zur Herstellung von Isocyanaten geeignet. Andere Herstellungsverfahren für N-substituierte Urethane gewinnen daher zunehmend an Bedeutung.

So beschreibt die DE-OS 2 160 111 ein Verfahren zur Herstellung von N-substituierten Urethanen durch Umsetzung eines organischen Carbonats mit einem primären oder sekundären Amin in Gegenwart einer Lewissäure. Nachteilig an diesem Verfahren ist, dass die Umsetzungsgeschwindigkeiten recht gering und die Reaktionszeiten dementsprechend gross sind; ausserdem werden als Nebenprodukte zusätzlich stets N-Alkyl-arylamine erhalten.

Nach Angaben der US-PS 2 834 799 werden Carbamidsäure- und Kohlensäureester durch Umsetzung von Harnstoffen mit Alkoholen in Gegenwart von Bortrifluorid hergestellt. Nachteilig ist hierbei dass das Bortrifluorid als Katalysator in äquimolaren Mengen benötigt wird, so dass pro erzeugtes Molekül Carbamidsäureester mindestens ein und pro Molekül Kohlensäureester mindestens zwei Moleküle Bortrifluorid verbracht werden. Dadurch gestaltet sich das Verfahren nicht nur teuer, sondern es stellt auch eine erhebliche Umweltbelastung dar, da das Bortrifluorid in Form des $H_3N \cdot BF_3$-Adduktes anfällt.

Methyl-N-phenylurethan kann ferner aus Kohlenmonoxid, Schwefel, Anilin und Methanol hergestellt werden (R.A. Franz et al., J. Org. Chem. 28, 585 [1963]). Nachteilig sind hierbei insbesondere die geringen Ausbeuten, die auch bei langen Reaktionszeiten nicht über 25% liegen.

Nach Angabe der US-PS 2 409 712 können N-Alkyl- und N-Arylurethane durch Umsetzung von Monoaminen mit Harnstoff, N,N′-Dialkyl- oder N,N′-Diarylharnstoff und Alkoholen bei Temperaturen von 150° bis 350°C, gegebenenfalls unter erhöhtem Druck, hergestellt werden. Beispielhaft beschrieben wird jedoch lediglich die Herstellung von N-Alkylmonourethanen nach der genannten Methode, während die Herstellung von N,N′-disubstituierten Diurethanen und von Polyurethanen nicht erwähnt wird.

Zur Herstellung von N-substituierten Monourethanen wird der Harnstoff gemäss US-PS 2 677 698 zunächst mit Monoaminen in den entsprechenden N,N′-disubstituierten Harnstoff übergeführt, dieser wird gereinigt und anschliessend mit Alkohol zur Reaktion gebracht.

Nachteilig an den genannten Verfahren sind neben einer aufwendigen Technik insbesondere die geringen Ausbeuten, die auch durch die Herstellung und Reinigung von N,N′-disubstituierten Harnstoffen nicht verbessert werden können.

Diese Nachteile können auch mit Hilfe des Verfahrens nach US-PS 2 806 051 nicht beseitigt werden. Nach Angaben dieser Patentschrift wird beispielsweise n-Hexylamin mit Harnstoff und Alkohol im Molverhältnis 1,0 : 1,2 : 2,0 bei Temperaturen unter 200°C, vorzugsweise von 120° bis 160°C, umgesetzt. Auch in den vorzugsweise genannten Temperaturbereichen werden in technisch zweckmässigen Reaktionszeiten nach dieser Verfahrensweise N-substituierte Urethane nur in geringen Ausbeuten erhalten. Es ist daher nicht überraschend, dass in der nachgängigen US-PS 3 076 007 zur Herstellung bon N-Alkyl- und N-Cycloalkylurethanen die obengenannten Methoden unter Verwendung von gegebenenfalls substituierten Harnstoffen nicht beschrieben werden; erwähnt wird hingegen die Umsetzung von Phosgen mit Alkoholen zu Chloralkylformiaten und die anschliessende Weiterreaktion mit Aminen zu Urethanen, während als besonders vorteilhaft zur Herstellung der Urethane die Reaktion von Aminen mit Äthylencarbonat empfohlen wird.

Verfährt man analog den Angaben der Beispiele der US-PS 2 806 051, setzt jedoch anstelle von Monoaminen Diamine, beispielsweise Hexamethylen-Diamin um, so erhält man dem beschriebenen Stand der Technik entsprechend mit hoher Ausbeute in Form eines Niederschlags ein Produkt, dessen Struktur weitgehend identisch ist mit den bekannten Polyharnstoffen aus Diaminen und Polyisocyanaten. Dies verdeutlicht ebenfalls, dass das Verfahren gemäss US-PS 2 806 051 auf die Umsetzung von Monoaminen beschränkt ist. Es ist daher nicht überraschend, dass in den genannten Patentschriften die Herstellung von Di- und/oder Polyurethanen, ausgehend von aliphatischen und/oder cycloaliphatischen Di- und/ oder Polyaminen, nach diesem Verfahrensprinzip nicht erwähnt wird.

Die Aufgabe der vorliegenden Erfindung bestand darin, aliphatische und/oder cycloaliphatische Di- und/oder Polyurethane aus leicht zugänglichen Ausgangskomponenten, möglichst in einer Reaktionsstufe unter wirtschaftlich vertretbaren Bedingungen in guten Ausbeuten herzustellen. Die Verwendung von stark toxischen Ausgangsstoffen, beispielsweise von Phosgen, Kohlenmonoxid oder Katalysatoren, die toxisch sind oder im Laufe der Umsetzung toxische Verbindungen bilden, beispielsweise Schwefelwasserstoff, sollte weitgehend vermieden werden.

Diese Aufgabe wurde gelöst durch ein Verfahren zur Herstellung von aliphatischen und/oder cycloaliphatischen Di- und/oder Polyurethanen, das dadurch gekennzeichnet ist, dass man ein Amin der Formel $R-(NH_2)_n$, in der R einen mehrwertigen gegebenenfalls substituierten aliphatischen Rest mit 2 bis 20 Kohlenstoffatomen, welcher eingeschobene Heteroatome oder zweiwertige heterocyclische Reste als Brückenglieder gebunden enthalten kann, einen cycloaliphatischen Rest mit 5 bis 12 Kohlenstoffatomen oder einen aliphatisch-cycloaliphatischen Rest mit 8 bis 50 Kohlenstoffatomen und n eine ganze Zahl von wenigstens 2 und nicht mehr als 6 bedeuten, mit Harnstoff und einem gegebenenfalls substituierten primären aliphatischen Monoalkohol mit 1 bis 20 Kohlenstoffatomen, einen sekundären aliphatischen oder cycloaliphatischen Monoalkohol mit 3 bis 15 Kohlenstoffatomen oder aromatisch-aliphatischen Monoalkohol gegebenenfalls in Gegenwart von Katalysatoren in solchen Mengen bei Temperaturen von 160 bis 300°C umsetzt, dass das Verhältnis von $NH_2$-Gruppen der primären aliphatischen und/oder cycloaliphatischen Di- und/oder Polyamine zu Harnstoff zu Hydroxylgruppen oder Alkohole 1 : 0,7 bis 10 : mindestens 1,25 bis höchstens 50 beträgt und gegebenenfalls den entstehenden Ammoniak abtrennt.

Die Reaktion kann durch folgende Gleichung veranschaulicht werden:

$$R-(NH_2)_n + nH_2NCONH_2 + HOR' \xrightarrow{\Delta} (NHCOOR')_n + 2nNH_3$$

Die Bildung von aliphatischen und cycloaliphatischen Di- und/oder Polyurethanen, insbesondere in einem Verfahrensschritt und in guten Ausbeuten ist besonders überraschend, da nach bekannter Lehrmeinung aus Diaminen und Harnstoff die entsprechenden Diharnstoffe, beispielsweise aus Hexamethylendiamin und Harnstoff Hexamethylen-diharnstoff, erhalten wird. Aus Harnstoff und Alkohol können auch Urethane erhalten werden, die jedoch in Gegenwart von Aminen zu N,N'-disubstituierten Harnstoffen weiterreagieren (Methoden der organischen Chemie, Houben-Weyl, Band 8, Seiten 151 und 140, Georg Thieme Verlag, Stuttgart, 1952). Anstelle von Diharnstoffen können ferner gemäss DE-PS 896 412 aus Diamiden der Kohlensäure, z.B. Harnstoff und Diaminen, deren Aminogruppen durch eine Kette von mehr als 3 Atomen getrennt sind, hochmolekulare, verspinnbare Kondensationsprodukte hergestellt werden. Hochmolekulare Polyharnstoffe, beispielsweise mit Molekulargewichten von 8000 bis 10 000 und grösser, werden auch erhalten, wenn man Diurethane mit Diaminen bei Temperaturen von ungefähr 150°C bis 300°C kondensiert (US-PS 2 181 663 und US-PS 2 568 885). Da Mono- und Polyurethane ausserdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte z.B. Biurete, Allophanate, Isocyanurate, Polycarbodiimide u.a. bilden können (J.A.C.S. 80, 5495 [1958] und J.A.C.S. 1946 [1956]) konnte nicht erwartet werden, dass unter sehr ähnlichen Reaktionsbedingungen nach dem erfindungsgemässen Verfahren aliphatische und/oder cycloaliphatische Di- und/oder Polyurethane in sehr guten Ausbeuten erhalten werden. Dies war besonders überraschend, da Versuche zur Herstellung von Diurethanen aus den obengenannten Produkten nach den erfindungsgemässen Reaktionsbedingungen nicht zum Ziele führten.

Für die erfindungsgemässe Umsetzung mit Harnstoff und Alkoholen eignen sich Amine der Formel $R-(NH_2)_n$, worin R einen mehrwertigen, gegebenenfalls substituierten aliphatischen oder cycloaliphatischen Rest oder gemischte Reste dieser Art und n eine ganze Zahl bedeuten, deren Wert der Valenzzahl von R entspricht und wenigstens 2, vorzugsweise 2 bis 5 und insbesondere 2 beträgt. Die aliphatischen Reste enthalten 2 bis 20, vorzugsweise 3 bis 16 und insbesondere 4 bis 12 Kohlenstoffatome; sie können eine geradkettige oder verzweigte Struktur besitzen und eingeschobene Heteroatome, z.B. Sauerstoff, Schwefel oder ein tertiäres Stickstoffatom, oder zweiwertige heterocyclische Reste als Brückenglieder gebunden enthalten. Die cycloaliphatischen Reste enthalten 5 bis 12, vorzugsweise 6 bis 12 Kohlenstoffatome, während die gemischten Reste dieser Art 8 bis 50, vorzugsweise 10 bis 15 Kohlenstoffatome aufweisen. Im einzelnen seien beispielhaft genannt:

aliphatische Diamine, wie Äthylendiamin, 1,3- und 1,2-Propylendiamin, 2,2-Dimethyl-1,3-propylendiamin, 1,4-Butylendiamin, 1,5-Pentamethylen-diamin, 1,6-Hexamethylen-diamin, 2,2,4-Trimethyl-1,6-hexamethylen-diamin, 1,8-Octamethylendiamin, 1,10-Decyclendiamin und 1,12-Dodecylen-diamin, cycloaliphatische Diamine, wie 1,2-, 1,3- und 1,4-Cyclohexan-diamin, 2,4- und 2,6-Hexahydrotoluylen-diamin sowie die entsprechenden Isomerengemische, aliphatisch-cycloaliphatische Diamine, wie 1,4-Hexahydroxylylendiamin,4,4'-, 2,4'- und 2,2'-Diamino-dicyclohexylmethan sowie die entsprechenden Isomerengemische 2,2-Di-(4-aminocyclohexyl)-propan, 3-Aminomethyl-3,5,5-trimethylcyclohexylamin, und Dicyclopentadienylverbindungen der Formel

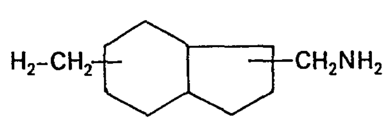

Polyamine wie Polycyclohexyl-polymethylen-polyamine der Formel

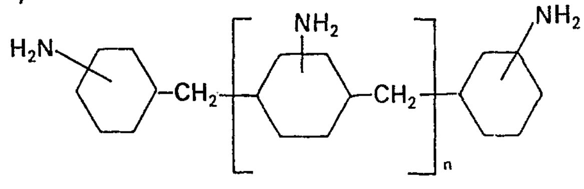

wobei n = 1 bis 4 ist und Mischungen aus Diamino-dicyclohexylmethanen und Polycylohexyl-polymethylen-polyaminen und Heteroatome oder heterocyclische Reste gebunden enthaltende Diamine, wie 3,3'-Diamino-dipropyläther, gegebenenfalls substituierte N,N'-Bis(aminoalkyl)piperazine, z.B. N,N'-Bis(2,2-dimethyl-3-aminopropyl)piperazin und N,N'-Bis(aminopropyl)piperazin. Werden derartige Diurethane nach konventionellen Methoden, beispielsweise mit Phosgen oder Chlorkohlensäureester hergestellt, so fallen als Nebenprodukte beträchtliche Salzmengen an.

Besonders bewährt haben sich und daher vorzugsweise verwendet werden 1,6-Hexamethylendiamin, 2,2,4-Trimethyl-1,6-hexamethylen-diamin, 1,4-Hexahydroxylylen-diamin, 2,4- und 2,6-Hexahydrotoluylen-diamin sowie die entsprechenden Isomerengemische, 4,4'-Diamino-dicyclohexyl-methan, 1,4-Diamino-cyclohexan, 2,2-Di-(4-aminocylohexyl)-propan und 3-Aminomethyl-3,5,5-trimethyl-cyclohexylamin.

Als Alkohole für das erfindungsgemässe Verfahren können beliebige, gegebenenfalls substituierte primäre oder sekundäre aliphatische Alkohole und aromatisch-aliphatische Alkohole sowie Mischungen davon verwendet werden. In Betracht kommen beispielsweise primäre aliphatische Monoalkohole mit 1 bis 20 Kohlenstoffatomen, vorzugsweise 1 bis 10 Kohlenstoffatomen, wie Methanol, Äthanol, Propanol, n-Butanol, Isobutanol, 2- und 3-Methylbutanol, Neopentylalkohol, Pentanol, 2-Methyl-pentanol, n-Hexanol, 2-Äthylhexanol, n-Heptanol, n-Octanol, n-Nonanol, n-Decanol, n-Dodecanol, 2-Phenylpropanol und Benzylalkohol, sekundäre aliphatische und cycloaliphatische Monoalkohole mit 3 bis 15 Kohlenstoffatomen, vorzugsweise 3 bis 6 Kohlenstoffatomen, wie Isopropanol, sec-Butanol, sec-Isoamylalkohol, Cyclopentanol, Cyclohexanol, 2,3- oder 4-Methyl-cyclohexanol und 4-tert.-Butyl-cyclohexanol. Vorzugsweise verwendet werden die Monoalkohle Methanol, Äthanol, Propanol, Butanol, Isobutanol, 2- und 3-Methyl-butanol, 2-Äthyl-butanol, Pentanol, 2-Methylpentanol, Hexanol, 2-Äthylhexanol, Heptanol, Octanol und Cyclohexanol.

Zur Herstellung der aliphatischen und/oder cycloaliphatischen Di- und/oder Polyurethane nach dem erfindungsgemässen Verfahren werden die obengenannten primären aliphatischen, cycloaliphatischen und/oder aliphatisch-cycloaliphatischen Di- und/oder Polyamine und Alkohole mit dem Harnstoff in solchen Mengen zur Reaktion gebracht, dass das Verhältnis von $NH_2$-Gruppen der Amine zu Harnstoff zu Hydroxylgruppen der Alkohole 1 : 0,7–10 : 1,25–50, vorzugsweise 1 :0,9–2,5 : 1,25–15 und insbesondere 1 : 1–2 : 1,25–10 beträgt.

Die Umsetzung wird vorzugsweise in Gegenwart von überschüssigem Alkohol als Lösungs- und Reaktionsmittel und in Abwesenheit oder gegebenenfalls in Gegenwart von Katalysatoren bei erhöhten Temperaturen und gegebenenfalls unter vermindertem oder erhöhtem Druck durchgeführt, wobei es sich als vorteilhaft erwiesen hat, den entstehenden Ammoniak sofort aus der Reaktionsmischung, beispielsweise durch Destillation, abzutrennen.

Wie bereits dargelegt wurde, wird die Umsetzung vorzugsweise in einem Alkoholüberschuss durchgeführt, so dass der Alkohol als Reaktionskomponente und gleichzeitig als Lösungsmittel fungiert. Anstelle von Alkohol können jedoch auch Mischungen aus Alkoholen und anderen unter den Reaktionsbedingungen inerten organischen Lösungsmitteln verwendet werden.

Die Herstellung der aliphatischen und/oder cycloaliphatischen Di- und/oder Polyurethane, vorzugsweise der Diurethane, wird erfindungsgemäss zweckmässigerweise in Abwesenheit von Katalysatoren durchgeführt, da üblicherweise die Umsetzung in wirtschaftlich tragbaren Reaktionszeiten mit guten Ausbeuten erfolgt. Auf diese Weise werden kostspielige Reinigungsoperationen zur Abtrennung der Katalysatoren aus den erhaltenen Endprodukten vermieden.

Wird zur Erhöhung der Reaktionsgeschwindigkeit, vorzugsweise bei niedrigen Temperaturen, die Umsetzung in Gegenwart von Katalysatoren durchgeführt, so werden diese zweckmässigerweise in Mengen von 0,1 bis 20 Gew.%, vorzugsweise 0,5 bis 10 Gew.% und insbesondere 1 bis 5 Gew.%, bezogen auf das Gewicht des primären Di- oder Polyamins, verwendet. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäss Handbook of Chemistry and Physics 14th. Edition, publiziert von Chemical Rubber Publishing Co, 2310 Superior Ave., N.E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumäthanolat, Lithiumpropanolat, Lithiumbutanolat, Natriummethanolat, Kalium-tert.-butanolat, Magnesiummethanolat, Calciummethanolat, Zinn(II)-chlorid, Zinn(IV)-chlorid, Bleiacetat, Bleiphosphat, Antimon(III)-chlorid, Antimon(V)-chlorid, Aluminiumisobutylat, Aluminiumtrichlorid, Wismut(III)-chlorid, Kupfer(II)-acetat, Kupfer(II)-sulfat, Kupfer(II)-nitrat, Bis-(triphenylphosphinoxido)-kupfer(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer(IV)-oxid, Uranylacetat, Titan-tetrabutanolat, Titantetrachlorid, Titanetraphenolat, Titannaphthenat, Vanadium(III)-chlorid, Vanadiumacetonylacetat, Chrom(III)-chlorid, Molybdän(VI)-oxid, Molybdänacetylacetonat, Wolfram(VI)-oxid, Mangan(II)-chlorid, Mangan(II)-acetat, Mangan(III)-acetat, Eisen(II)-acetat, Eisen(III)-acetat, Eisenphosphat, Eisenoxalat, Eisen(III)-chlorid, Eisen(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Umsetzung wird bei Temperaturen von 160 bis 300°C, vorzugsweise von 180 bis 250°C und insbesondere von 185 bis 230°C und Drücken von 0,1 bis 120 bar, vorzugsweise 0,5 bis 60 bar, insbesondere 1 bis 40 bar durchgeführt. Für diesen Temperaturbereich ergeben sich Reaktionszeiten von 0,1 bis 50 Stunden, vorzugsweise von 1 bis 20 Stunden und insbesondere 4 bis 12 Stunden. Bei gegebener Temperatur wird die Reaktion dann vorzugsweise unter einem Druck durchgeführt, bei dem der entstehende Ammoniak selektiv aus dem Reaktionsgemisch abdestilliert werden kann. Die entsprechenden Werte können Tabellen mit physikalischen Kenndaten des Ammoniak und der Alkohole entnommen werden.

Nach dem erfindungsgemässen Verfahren werden die Di- und/oder Polyurethane zweckmässig wie folgt hergestellt: Die Ausgangskomponenten werden in den entsprechenden Molverhältnissen gemischt und gegebenenfalls in Gegenwart von Katalysatoren in einem Reaktionsgefäss oder gegebenenfalls einem Druckgefäss, ausgestattet mit einer Vorrichtung zur Abtrennung des Ammoniaks, erhitzt. Der entstehende Ammoniak kann nach beendeter Umsetzung abgetrennt werden: vorzugsweise wird er jedoch bereits im Laufe der Umsetzung abdestilliert. Hierbei kann es zweckmässig sein, insbesondere bei der Umsetzung von niedermolekularen Alkoholen unter Druck, den Ammoniak mit Hilfe eines unter den Reaktionsbedingungen inerten Strippmittels, beispielsweise eines Gases, wie Stickstoff, abzutrennen.

Nach einer besonders vorteilhaften Ausführungsform, die in der Regel zu einer erheblichen Reduzierung der Reaktionszeit führt, werden die primären aliphatischen und/oder cycloaliphatischen Di- und/oder Polyamine, der Harnstoff und Alkohol zunächst in einem Verhältnis $NH_2$-Gruppen der Amine zu Harnstoff zu Alkohol von 1 : 1–1,5 : 1–2, vorzugsweise 1 : 1–1,25 : 1,25–1,75 1 bis 4 Stunden, vorzugsweise 2 bis 3 Stunden zur Reaktion gebracht, danach wird der Reaktionsmischung zusätzlicher Alkohol in einer solchen Menge einverleibt, dass pro eingesetzter $NH_2$-Gruppen der Amine 2,5 bis 7,5, vorzugsweise 3 bis 6 Mol Alkohol vorliegen, und die Reaktion in einem Zeitraum von insgesamt 4 bis 20 Stunden, vorzugsweise 5 bis 12 Stunden zu Ende geführt. Aus der erhaltenen Reaktionsmischung wird anschliessend, gegebenenfalls nach Abtrennung des Katalysators und/oder Abfiltrieren von Feststoffen, das Di- und/oder Pulyurethan isoliert, beispielsweise durch Abdestillieren des Alkohols und/oder des Lösungsmittels sowie gegebenenfalls von als Nebenprodukt gebildeten 0-Alkylcarbamaten, durch partielles Abdestillieren des Alkohols und Auskristallisieren, durch Auskristallisieren, durch Ausfällen mit oder auch durch Umkristallisieren aus anderen Lösungsmitteln.

Die nach dem erfindungsgemässen Verfahren hergestellten aliphatischen und/oder cycloaliphatischen Di- und/oder Polyurethane sind wertvolle End- und Zwischenprodukte. Sie werden beispielsweise als Schädlingsbekämpfungsmittel verwendet. Als Zwischenprodukte werden sie als Komponenten für Polykondensations- und Polymersysteme eingesetzt, insbesondere jedoch unter Abspaltung von Alkohol in die entsprechenden Isocyanate übergeführt, wobei Di- und Polyisocyanate zur Herstellung von Polyurethanen Anwendung finden.

Die in den Beispielen genannten Teile beziehen sich auf das Gewicht. Die Elementarzusammensetzungen und Strukturen sind durch Elementaranalyse, Massenspektrometrie sowie IR- und NMR-Spektren bestätigt.

Beispiel 1

116 Teile 1,6-Hexamethylendiamin werden mit 120 Teilen Harnstoff und 1300 Teilen n-Octanol-(1) unter kräftigem Rühren und Abdestillieren von Ammoniak 20 Stunden auf Rückflusstemperatur (185–200°C Normaldruck) erhitzt. Das Reaktionsprodukt kristallisiert beim Abkühlen des Reaktionsgemisches aus. Man erhält durch Filtrieren und Trocknen 389 Teile 1,6-Di-(octoxicarbonyl-amino)-hexan, $C_{24}H_{48}N_2O_4$ (Molekulargewicht 428), entsprechend 91% der Theorie, bezogen auf die Einsatzstoffe 1,6-Hexamethylendiamin und Harnstoff. Schmelzpunkt 108–109°C.

Beispiel 2

116 Teile 1,6-Hexamethylendiamin werden mit 120 Teilen Harnstoff, 1,5 Teilen Natrium-octylat und 1300 Teilen n-Octanol-(1) analog Beispiel 1 16 Stunden auf Rückflusstemperatur erhitzt. Man erhält nach Abkühlen, Filtrieren, Waschen mit n-Octanol-(1) und Trocknen 396 Teile 1,6-Di(octoxicarbonyl-amino)-hexan, entsprechend 92,5% der Theorie. Schmelzpunkt 106–108°C.

Beispiel 3

116 Teile 1,6-Hexamethylendiamin werden mit 132 Teilen Harnstoff und 1300 Teilen n-Octanol-(1) gemäss Beispiel 1 umgesetzt. Man erhält 398 Teile 1,6-Di-(octoxicarbonyl-amino)-hexan, entsprechend 93% der Theorie, bezogen auf 1,6-Hexamethylendiamin. Schmelzpunkt 107–109°C.

Beispiel 4

116 Teile 1,6-Hexamethylendiamin werden mit

132 Teilen Harnstoff und 390 Teilen n-Octanol-(1) 10 Stunden bei Rückflusstemperatur (185–200°C) gerührt. Man filtriert von dem auf etwa 100°C abgekühlten Reaktionsgemisch einen ungelösten Feststoff ab, lässt das Reaktionsprodukt durch Abkühlen auf Raumtemperatur auskristallisieren und erhält nach Filtrieren, Waschen mit n-Octanol-(1) und Trocknen 320 Teile 1,6-Di-(octoxicarbonyl-amino)-hexan, entsprechend 77% der Theorie, bezogen auf 1,6-Hexamethylendiamin (Reinheit ca. 95%). Schmelzpunkt 104–106°C.

Beispiel 5

116 Teile 1,6-Hexamethylendiamin werden mit 132 Teilen Harnstoff und 390 Teilen n-Octanol-(1) 2 Stunden bei Rückflusstemperatur (185–200°C) gerührt. Dann lässt man zusätzlich 520 Teile n-Octanol-(1) in das Reaktionsgemisch einfliessen und führt die Umsetzung in 8 Stunden bei Rückflusstemperatur zu Ende. Man lässt das Reaktionsprodukt durch Abkühlen auskristallisieren und erhält nach dem Filtrieren, Waschen mit n-Octanol-(1) und Trocknen 411 Teile 1,6-Di-(octoxicarbonyl-amino)-hexan, entsprechend 96% der Theorie, bezogen auf 1,6-Hexamethylendiamin. Schmelzpunkt 107–108°C.

Beispiel 6

232 Teile 1,6-Hexamethylendiamin werden mit 264 Teilen Harnstoff und 450 Teilen n-Butanol unter Durchsatz von 10 l Stickstoff je Liter Reaktionsgemisch und Stunde über ein Tauchrohr 3 Stunden bei Rückflusstemperatur (185–195°C) und 6–7 bar gerührt. Dann lässt man zusätzlich 450 Teile n-Butanol in das Reaktionsgemisch einfliessen und setzt die Umsetzung 8 Stunden bei 195–200°C (7 bar) fort. Man lässt das Reaktionsprodukt durch Abkühlen des Reaktionsgemisches auskristallisieren und erhält nach Filtrieren und Umkristallisieren aus Aceton/Wasser 594 Teile 1,6-Di-(butoxicarbonyl-amino)-hexan, $C_{16}H_{32}N_2O_4$ (Molekulargewicht 316), entsprechend 94% der Theorie, bezogen auf 1,6-Hexamethylendiamin. Schmelzpunkt 90–92°C.

Beispiel 7

116 Teile 1,6-Hexamethylendiamin werden mit 120 Teilen Harnstoff und 130 Teilen Äthanol unter Durchsatz von 7 l Stickstoff je Liter Reaktionsgemisch und Stunde über ein Tauchrohr 2 Stunden bei Rückflusstemperatur (185–195°C) und 23–25 bar gerührt. Dann lässt man zusätzlich 200 Teile Äthanol in das Reaktionsgemisch einfliessen und setzt die Umsetzung 8 Stunden bei 195–200°C fort. Man engt das Reaktionsgemisch durch Abdestillieren von Äthanol ein, filtriert das auskristallisierende Produkt und erhält nach Umkristallisieren aus Aceton/Wasser 227 Teile Di-(äthoxycarbonyl-amino)-hexan, $C_{12}H_{24}N_2O_4$ (Molekulargewicht 260), entsprechend 87% der Theorie, bezogen auf 1,6-Hexamethylendiamin. Schmelzpunkt 80–82°C.

Beispiel 8

210 Teile 4,4'-Diamino-dicyclohexyl-methan werden mit 132 Teilen Harnstoff und 390 Teilen n-Octanol-(1) 2 Stunden bei Rückflusstemperatur (185–205°C) und unter Abdestillieren von Ammoniak gerührt. Dann lässt man zusätzlich 510 Teile n-Octanol-(1) einfliessen und setzt die Umsetzung 10 Stunden bei Rückflusstemperatur fort. Man filtriert das auf 100°C abgekühlte Reaktionsgemisch, lässt das Reaktionsprodukt durch Abkühlen auf Raumtemperatur auskristallisieren und erhält nach Filtrieren, Waschen mit Octanol und Trocknen 494 Teile Di-(4-octoxi-carbonylaminocyclohexyl)-methan, $C_{31}H_{58}N_2O_4$ (Molekulargewicht 522), entsprechend 94,6% der Theorie, bezogen auf 4,4'-Diamino-dicyclohexyl-methan. Schmelzpunkt 129–131°C (aus Äthylacetat).

Vergleichsbeispiel

(in Analogie zur Herstellung von Monourethanen gemäss US 2 806 051)

116 Teile 1,6-Hexamethylendiamin werden mit 144 Teilen Harnstoff und 150 Teilen n-Butanol 20 Stunden bei 120–150°C und 1–3 bar gerührt. Es scheiden sich beträchtliche Mengen eines amorphen Feststoffs ab, dessen IR-Spektrum (nach Filtrieren und Trocknen) weitgehend deckungsgleich ist mit dem Spektrum von Poly-hexamethylen-harnstoff. Er löst sich beim Erhitzen in n-Butanol nach 2 Stunden bei 190° und 6–7 bar nicht auf. Aus dem Filtrat des Reaktionsgemisches konnte kein Di-(butoxicarbonylamino)-hexan isoliert werden.

Beispiel 9

142 Teile 1,4-Hexahydro-xylylen-diamin werden mit 126 Teilen Harnstoff und 400 Teilen n-Octanol-(1) 2 Stunden bei Rückflusstemperatur (185–200°C) unter Abdestillieren von Ammoniak gerührt. Dann lässt man zusätzlich 600 Teile n-Octanol-(1) in das Reaktionsgemisch einfliessen und setzt die Umsetzung 8 Stunden bei Rückflusstemperatur fort. Man filtriert das auf 110°C abgekühlte Reaktionsgemisch, lässt das Reaktionsprodukt durch Abkühlen auf Raumtemperatur auskristallisieren und erhält nach Filtrieren, Waschen mit n-Octanol-(1) und Trocknen 434 Teile 1,4-Di-(octoxicarbonyl-aminomethyl)-cyclohexan, $C_{26}H_{50}N_2O_4$ (Molekulargewicht 454), entsprechend 95,4% der Theorie, bezogen auf 1,4-Hexahydro-xylylendiamin. Schmelzpunkt 122°C (aus Äthylacetat).

Beispiel 10

114 Teile 1,4-Diamino-cyclohexan werden mit 120 Teilen Harnstoff und 390 Teilen n-Octanol-(1) 3 Stunden bei Rückflusstemperatur (185–200°C) und unter Abdestillieren von Ammoniak gerührt. Dann lässt man zusätzlich 610 Teile n-Octanol-(1) in das Reaktionsgemisch einfliessen und setzt die Umsetzung 7 Stunden bei Rückflusstemperatur fort. Man lässt das Reaktionsprodukt durch Abkühlen auskristallisieren und erhält nach Filtrieren, Waschen mit Octanol-(1) und Trocknen 391 Teile 1,4-Di-(octoxi-carbonyl-amino)-cyclohexan, $C_{24}H_{46}N_2O_4$ (Molekulargewicht 426), entspre-

chend 91,8% der Theorie, bezogen auf 1,4-Diamino-cyclohexan.

Beispiel 11

116 Teile Hexamethylendiamin werden mit 135 Teilen Harnstoff und 1300 Teilen Hexadecanol-(1) 18 Stunden unter Abdestillieren von Ammoniak bei 195–205°C gerührt. Man lässt das Reaktionsgemisch abkühlen, digeriert mit 2000 Teilen eines Gemisches Äthanol/Aceton (1/1) und filtriert. Als Rückstand verbleiben 411 Teile 1,6-Di-(hexadecoxi-carbonyl-amino)-hexan, $C_{40}H_{80}N_2O_4$ (Molekulargewicht 652), entsprechend 63% der Theorie, bezogen auf 1,6-Hexamethylendiamin. Schmelzpunkt 114–116°C (aus Äthylacetat). Das Filtrat enthält einen zusätzlichen Anteil 1,6-Di-(hexadecoxi-carbonyl-amino)-hexan.

Beispiel 12

116 Teile 1,6-Hexamethylendiamin werden mit 132 Teilen Harnstoff und 350 Teilen 2-Butoxiäthanol-(1) bei Rückflusstemperatur (180–200°C) und unter Abdestillieren von Ammoniak gerührt. Dann lässt man zusätzlich 650 Teile 2-Butoxiäthanol-(1) in das Reaktionsgemisch einfliessen und setzt die Umsetzung 8 Stunden bei Rückflusstemperatur fort. Man destilliert das nicht umgesetzte Butoxi-äthanol im Wasserstrahlvakuum weitgehend ab, nimmt den Rückstand in Methanol auf und fällt das Produkt durch Zusatz von Wasser. Man erhält nach Filtrieren und Trocknen 378 Teile 1,6-Di-(2-butoxi-äthoxi-carbonyl-amino)-hexan, $C_{20}H_{40}N_2O_6$ (Molekulargewicht 404), entsprechend 93,6% der Theorie, bezogen auf 1,6-Hexamethylendiamin. Schmelzpunkt 64–66°C.

Beispiel 13

156 Teile Di-(3-aminopropyl)-äther werden mit 132 Teilen Harnstoff und 390 Teilen n-Octanol-(1) unter Abdestillieren von Ammoniak 2 Stunden bei Rückflusstemperatur (185–200°C) gerührt. Dann lässt man zusätzlich 610 Teile n-Octanol in das Reaktionsgemisch einfliessen und setzt die Umsetzung 6 Stunden bei Rückflusstemperatur fort. Man destilliert nicht umgesetztes Octanol und O-Octylcarbamat bis zu einer Sumpftemperatur von ca. 180°C bei 2 mbar ab und erhält einen beim Erkalten kristallisierenden Rückstand von 432 Teilen Di-(3-octoxicarbonyl-amino-propyl)äther, $C_{24}H_{48}N_2O_5$ Molekulargewicht 444), entsprechend 97,3% der Theorie, bezogen auf Di-(3-aminopropyl-äther. (Reinheit ca. 96%). Schmelzpunkt 61°C (aus Äthylacetat).

Beispiel 14

256 Teile N,N'-Bis-(2,2-dimethyl-3-aminopropyl)-piperazin werden mit 126 Teilen Harnstoff und 1300 Teilen n-Octanol-(1) unter Abdestillieren von Ammoniak 16 Stunden bei Rückflusstemperatur (185–200°C) gerührt. Man destilliert nicht umgesetztes Octanol und O-Octylcarbamat bis zu einer Sumpftemperatur von 180–200°C bei 2 mbar rasch ab und erhält als Rückstand 559 Teile N,N'-Bis-(2,2-dimethyl-3-octoxicarbonyl-amino-propyl)-piperazin, $C_{32}H_{64}N_4O_4$ (Molekulargewicht 568), entsprechend 99% der Theorie, bezogen auf N,N'-Bis-(2,2-dimethyl-3-aminopropyl)-piperazin (Reinheit ca. 94%). Schmelzpunkt 67–68°C (aus Aceton/Wasser).

Beispiel 15

170 Teile 3-Aminomethyl-3,5,5-trimethyl-1-aminocyclohexan werden mit 120 Teilen Harnstoff und 400 Teilen n-Octanol unter Abdestillieren von Ammoniak 2 Stunden bei Rückflusstemperatur (185–200°C) gerührt. Dann lässt man zusätzlich 500 Teile n-Octanol in das Reaktionsgemisch einfliessen und setzt die Umsetzung 10 Stunden bei Rückflusstemperatur fort. Man engt das Reaktionsgemisch durch Destillation bis zu einer Sumpftemperatur von ca. 200°C bei 2 mbar rasch ein und erhält als partiell kristallisierenden Rückstand 463 Teile 3-(Octoxicarbonyl-amino-methyl)-3,5,5-trimethyl-1-(octoxicarbonyl-amino)-cyclohexan, $C_{28}H_{54}N_2O_4$ (Molekulargewicht 482), entsprechend 96% der Theorie (Reinheit ca. 95%).

Beispiel 16

116 Teile 1,6-Hexamethylendiamin werden mit 120 Teilen Harnstoff und 400 Teilen 2-Äthylhexanol unter Abdestillieren von Ammoniak 3 Stunden bei Rückflusstemperatur (185–190°C) gerührt. Dann fügt man weitere 800 Teile 2-Äthylhexanol zu und setzt die Umsetzung bei Rückflusstemperatur 15 Stunden fort. Man destilliert bis zu einer Sumpftemperatur von 190–200°C bei 2 mbar rasch ab und erhält als langsam kristallisierenden Rückstand 445 Teile 1,6-Di-(2-äthylhexoxi-carbonyl-amino)-hexan, $C_{24}H_{28}N_2O_4$ (Molekulargewicht 428) (Reinheit ca. 75%).

Beispiel 17

116 Teile 1,6-Hexamethylendiamin werden mit 120 Teilen Harnstoff und 1000 Teilen Cyclohexanol 18 Stunden unter Abdestillieren von Ammoniak bei Rückflusstemperatur (185–195°C) und 2–3 bar gerührt. Man lässt das Reaktionsgemisch auf ca. 100°C abkühlen, filtriert und engt das Filtrat durch Destillation bis zu einer Sumpftemperatur von ca. 180°C bei 5–10 mbar ein. Durch Kristallisation des Rückstands aus Methanol/Wasser erhält man 317 Teile 1,6-Di-(cyclohexoxicarbonyl-amino)-hexan, $C_{20}H_{36}N_2O_4$ (Molekulargewicht 368), entsprechend 86% der Theorie. Schmelzpunkt 98–100°C.

Beispiel 18

128 Teile 2,4-Hexahydro-toluylendiamin werden mit 144 Teilen Harnstoff und 1300 Teilen n-Octanol 18 Stunden unter Abdestillieren von Ammoniak bei Rückflusstemperatur (185–195°C) gerührt. Man destilliert aus dem Reaktionsgemisch n-Octanol und O-Octylcarbamat bis zu einer Sumpftemperatur von ca. 200°C bei 2–3 mbar rasch ab, nimmt den Rückstand in heissem Äthylacetat auf, filtriert den beim Abkühlen auskristallisierenden cyclischen Harnstoff des 2,4-Hexahydro-toluylen-diamins ab und erhält

nach Abdestillieren des Lösungsmittels als schwachgelben, wachsartigen Rückstand 264 Teile 2,4-Di-(octoxicarbonyl-amino)-methylcyclohexan, $C_{25}H_{48}N_2O_4$ (Molekulargewicht 440), entsprechend 60% der Theorie, bezogen auf 2,4-Hexahydro-toluylen-diamin (Reinheit ca. 94°C).

Beispiel 19

102 Teile Diamino-neopentan werden mit 180 Teilen Harnstoff und 1000 Teilen n-Octanol 18 Stunden unter Abdestillieren von Ammoniak bei Rückflusstemperatur (185–200°C) gerührt. Man destilliert aus dem Reaktionsgemisch n-Octanol und O-Octylcarbamat bis zu einer Sumpftemperatur von ca. 200°C bei 2–3 mbar rasch ab, nimmt den Rückstand in heissem Äthylacetat auf, filtriert das beim Abkühlen auskristallisierende 5,5-Dimethyl-hexahydropyrimidin-2-on ab und erhält nach Abdestillieren des Lösungsmittels als schwachgelben öligen Rückstand 198 Teile Di-(octoxicarbonyl-amino)-neopentan, $C_{23}H_{46}N_2O_4$ (Molekulargewicht 414), entsprechend 47,8% der Theorie, bezogen auf Diamino-neopentan (Reinheit ca. 95%).

Beispiel 20

5,8 Teile 1,6-Hexamethylendiamin werden mit 7,2 Teilen Harnstoff und 9,2 Teilen Äthanol unter Durchsatz von 7 Litern Stickstoff je Liter Reaktionsgemisch und Stunde über ein Tauchrohr 13 Stunden lang auf 170 bis 175°C erhitzt, wobei über ein Druckventil im Reaktionsgefäss ein Druck von 15 bis 16 bar eingestellt wird, so dass die Reaktionsmischung siedet. Nach dem Abkühlen untersucht man die Reaktionsmischung gaschromatographisch nach der Methode des «Internen Standard». Hierbei zeigt sich dass das 1,6-Hexamethylendiamin praktisch vollständig umgesetzt wurde, wobei 10,9 Teile (83,8% der Theorie, bezogen auf umgesetztes 1,6-Hexamethylendiamin) 1,6-Di-(äthoxycarbonylamino)-hexan entstanden sind. Dies entspricht einer Raum-Zeit-Ausbeute von 37,8 g/l • h.

Beispiele 21 bis 25

Man verfährt analog den Angaben von Beispiel 20, fügt der Reaktionsmischung jedoch zusätzlich 0,1 Teil eines Katalysators bei.

Die verwendeten Katalysatoren, die Reaktionszeiten und erhaltenen Ausbeuten sind in der folgenden Tabelle zusammengefasst.

Tabelle

| Beispiel | Katalysator | Zeit (h) | Ausbeute (%) | Raum-Zeit-Ausbeute (g/l·h) |
|---|---|---|---|---|
| 21 | Kobalt-(II)-acetat | 5,5 | 79,2 | 84,4 |
| 22 | Eisen-(II)-acetat | 5,0 | 73,1 | 85,5 |
| 23 | Vanadiumtrichlorid | 5,0 | 61,5 | 72,1 |
| 24 | Zinknaphthenat | 7,0 | 78,5 | 65,6 |
| 25 | Mangan-(II)-acetat | 7,0 | 82,4 | 68,9 |

**Patentansprüche**

1. Verfahren zur Herstellung von aliphatischen und/oder cycloaliphatischen Di- und/oder Polyurethanen, dadurch gekennzeichnet, dass man ein Amin der Formel $R-(NH_2)_n$, in der R einen mehrwertigen gegebenenfalls substituierten aliphatischen Rest mit 2 bis 20 Kohlenstoffatomen, welcher eingeschobene Heteroatome oder zweiwertige heterocyclische Reste als Brückenglieder gebunden enthalten kann, einen cycloaliphatischen Rest mit 5 bis 12 Kohlenstoffatomen oder einen aliphatisch-cycloaliphatischen Rest mit 8 bis 50 Kohlenstoffatomen und n eine ganze Zahl von wenigstens 2 und nicht mehr als 6 bedeuten, mit Harnstoff und einem gegebenenfalls substituierten primären aliphatischen Monoalkohol mit 1 bis 20 Kohlenstoffatomen, einen sekundären aliphatischen oder cycloaliphatischen Monoalkohol mit 3 bis 15 Kohlenstoffatomen oder aromatisch-aliphatischen Monoalkohol gegebenenfalls in Gegenwart von Katalysatoren in solchen Mengen bei Temperaturen von 160 bis 300°C umsetzt, dass das Verhältnis von $NH_2$-Gruppen der primären aliphatischen und/oder cycloaliphatischen Di- und/oder Polyamine zu Harnstoff zu Hydroxylgruppen der Alkohole 1 : 0,7 bis 10 : mindestens 1,25 bis höchstens 50 beträgt und gegebenenfalls den entstehenden Ammoniak abtrennt.

2. Verfahren gemäss Anspruch 1, dadurch gekennzeichnet, dass man gleichzeitig den entstehenden Ammoniak abtrennt.

3. Verfahren gemäss Anspruch 1, dadurch gekennzeichnet, dass man die Umsetzung bei Drukken von 0,1 bis 120 bar durchführt.

4. Verfahren gemäss Anspruch 1, dadurch gekennzeichnet, dass man als aliphatische Diamine 1,6-Hexamethylendiamin, als cycloaliphatische und aliphatisch-cycloaliphatische Diamine 1,4-Hexahydroxylylen-diamin 2,4- und 2,6-Hexahydrotoluylen-diamin sowie die entsprechenden Isomerengemische, 4,4′-Diamino-dicyclohexylmethan, 1,4-Diaminocyclohexan, 2,2-Di-(4-aminocyclohexyl)-propan und 3-Aminomethyl-3,5,5-trimethyl-cyclohexylamin verwendet.

5. Verfahren gemäss Anspruch 1, dadurch gekennzeichnet, dass man als Alkohole aliphatische und cycloaliphatische Alkohole verwendet.

6. Verfahren nach Anspruch 5, dadurch gekennzeichnet, dass man als Alkohole Methanol, Äthanol, Propanol, Butanol, Isobutanol, 2- und

3-Methylbutanol, 2-Äthylbutanol, Pentanol, 2-Methyl-pentanol, Hexanol, 2-Äthylhexanol, Heptanol, Octanol und Cyclohexanol verwendet.

7. Verfahren gemäss Anspruch 1, dadurch gekennzeichnet, dass man als Katalysatoren Verbindungen verwendet, die ein oder mehrere Kationen von Metallen aus den Gruppen IA, IB, IIA, IIB, IIIA, IIIB, IVA, IVB, VA, VB, VIB, VIIB und VIIIB des Periodensystems gebunden enthalten.

8. Verfahren gemäss Anspruch 1, dadurch gekennzeichnet, dass man die Ausgangskomponenten im Verhältnis $NH_2$-Gruppen der Amine zu Harnstoff zu Alkohol von 1:1–1,5:1–2 1 bis 4 Stunden lang kondensiert, der Reaktionsmischung zusätzlichen Alkohol in einer Menge einverleibt, dass das Verhältnis $NH_2$-Gruppen der Amine zu Alkohol 1 : 2,5–7,5 beträgt und die Reaktion zu Ende führt.

## Claims

1. A process for the production of aliphatic and/or cycloaliphatic di- and/or polyurethanes, wherein an amine of the formula $R-(NH_2)_n$, where R is a polyvalent, optionally substituted aliphatic radical of 2 to 20 carbon atoms which may contain interrupting heteroatoms or divalent heterocyclic radicals as bridge members in bonded form, a cycloaliphatic radical of 5 to 12 carbon atoms or an aliphatic-cycloaliphatic radical of 8 to 50 carbon atoms, and n is an integer of at least 2 but not more than 6, is reacted with urea and an optionally substituted primary aliphatic monoalcohol of 1 to 20 carbon atoms, a secondary aliphatic or cycloaliphatic monoalcohol of 3 to 15 carbon atoms or an aromatic-aliphatic monoalcohol in the presence or absence of a catalyst, at a temperature of from 160° to 300°C, in such amounts that the ratio of $NH_2$ groups of the primary aliphatic and/or cycloaliphatic di- and/or polyamines to urea to hydroxyl groups of the alcohol is 1 : 0.7 to 10 : at least 1.25 to not more than 50, and, if desired, the ammonia formed is separated off.

2. A process as claimed in claim 1, wherein the ammonia is separated off as it is formed.

3. A process as claimed in claim 1, wherein the reaction is carried out at a pressure of from 0.1 bar to 120 bars.

4. A process as claimed in claim 1, wherein 1,6-hexamethylenediamine is used as aliphatic diamine, and 1,4-hexahydroxylylenediamine, 2,4- and 2,6-hexahydrotoluenediamine, the corresponding isomer mixtures thereof, 4,4′-diaminodicyclohexylmethane, 1,4-diaminocyclohexane, 2,2-bis (4-aminocyclohexyl)-propane and 3-amino-methyl-3,5,5-trimethylcyclohexylamine are used as cycloaliphatic and aliphatic-cycloaliphatic diamines.

5. A process as claimed in claim 1, wherein aliphatic and cycloaliphatic alcohols are used as alcohols.

6. A process as claimed in claim 5, wherein methanol, ethanol, propanol, butanol, isobutanol, 2- and 3-methylbutanol, 2-ethylbutanol, pentanol, 2-methylpentanol, hexanol, 2-ethylhexanol, heptanol, octanol and cyclohexanol are used as alcohols.

7. A process as claimed in claim 1, wherein compounds which contain, in bonded form, one or more cations of metals selected from the group consisting of groups IA, IB, IIA, IIB, IIIA, IIIB, IVA, IVB, VA, VB, VIB, VIIB and VIIIB of the periodic system are used as catalysts.

8. A process as claimed in claim 1, wherein the starting components are condensed in a ratio of $NH_2$ groups of the amines to urea to alcohol of 1 : 1–1.5 : 1–2 for from 1 to 4 hours, and alcohol is added to the reaction mixture in such an amount that the ratio of $NH_2$ groups of the amines to alcohol is 1 : 2.5–7.5 and the reaction is completed.

## Revendications

1. Procédé de préparation de di- et/ou polyuréthanes aliphatiques et/ou cycloaliphatiques, caractérisé par le fait qu'on fait réagir, à des températures de 160 à 300°C, éventuellement en présence de catalyseurs, une amine de formule $R-(NH_2)_n$, dans laquelle R représente un reste aliphatique plurivalent, éventuellement substitué, ayant 2 à 20 atomes de carbone, qui peut contenir, liés, des hétéroatomes intercalés ou des restes cycloaliphatiques bivalents, comme termes pontaux, un reste cycloaliphatique à 5 à 12 atomes de carbone ou un reste aliphatique-cycloaliphatique à 8 à 50 atomes de carbone et n un nombre entier au moins égal à 2 et non supérieur à 6, avec une urée et un monoalcool aliphatique, primaire, éventuellement substitué, à 1 à 20 atomes de carbone, un monoalcool secondaire, aliphatique ou cycloaliphatique à 3 à 15 atomes de carbone ou un monoalcool aromatique aliphatique, en proportions telles que les rapports entre les groupes $NH_2$ des di- et/ou polyamides primaires aliphatiques et/ou cycloaliphatiques, l'urée et les groupes hydroxyle des alcools sont 1/0,7 à 10/ au moins 1,25 à au plus 50, et on sépare éventuellement l'ammoniac qui s'est formé.

2. Procédé selon la revendication 1, caractérisé par le fait qu'on sépare simultanément l'ammoniac formé.

3. Procédé selon la revendication 1, caractérisé par le fait qu'on effectue la réaction à des pressions de 0,1 à 120 bar.

4. Procédé selon la revendication 1, caractérisé en ce que l'on utilise, comme diamines aliphatiques, la 1,6-hexaméthylènediamine, comme diamines cycloaliphatique et aliphatique-cycloaliphatique, la 1,4-hexahydroxylylène-diamine, les 2,4- et 2,6-hexahydrotoluylène-diamines ainsi que les mélanges d'isomères correspondants, les 4,4′-diamino-dycyclohexylméthane, 1,4-diaminocyclohexane, 2,2-di-(4-aminocyclohexyl)-propane et 3-aminométhyl-3,5,5-triméthyl-cyclohexylamine.

5. Procédé selon la revendication 1, caractérisé par le fait que l'on utilise, comme alcools, des

alcools aliphatiques et cycloaliphatiques.

6. Procédé selon la revendication 5, caractérisé par le fait que l'on utilise, comme alcools, les méthanol, éthanol, propanol, butanol, isobutanol, 2- et 3-méthylbutanol, 2-éthylbutanol, pentanol, 2-méthyl-pentanol, hexanol, 2-éthylhexanol, heptanol, octanol et cyclohexanol.

7. Procédé selon la revendication 1, caractérisé par le fait qu'on utilise, comme catalyseurs, des composés qui contiennent, liés, un ou plusieurs cations de métaux des groupes IA, IB, IIA, IIB, IIIA, IIIB ,IVA, IVB, VA, VB, VIB, VIIB et VIIIB du système périodique.

8. Procédé selon la revendication 1, caractérisé par le fait que l'on condense pendant 1 à 4 heures les composants de départ dans les rapports, entre groupes $NH_2$ de l'amine, urée et alcool, de 1/1 à 1,5/1 à 2, on incorpore au mélange de réaction encore de l'alcool en quantité telle que le rapport, entre groupes $NH_2$ de l'amine et l'alcool, soit de 1/2,5 à 7,5 et on mène la réaction à sa fin.